# EUROPEAN PATENT SPECIFICATION

(11) **EP 1 928 383 B1**
(45) Date of publication and mention of the grant of the patent: **01.07.2009**
(21) Application number: 06803083.2
(22) Date of filing: 08.09.2006
(51) Int. Cl.: A61F 13/551

(54) **ABSORBENT PERSONAL CARE ARTICLE WITH A WRAP MEMBER HAVING DISTINCT COMPONENT LAYERS**
SAUGFÄHIGER KÖRPERPFLEGEARTIKEL MIT WICKELTEIL MIT VERSCHIEDENARTIGEN SCHICHTEN
ARTICLE D'HYGIÈNE PERSONNELLE ABSORBANT AVEC UNE ENVELOPPE POSSEDANT DES COUCHES D'ELEMENTS DISTINCTS

(30) Priority: 29.09.2005 US 241102
(43) Date of publication of application: 11.06.2008
(73) Proprietor: KIMBERLY-CLARK WORLDWIDE, INC., Neenah, WI 54956 (US)
(72) Inventor: THOMAS, Brian, Lee, Appleton, Wisconsin 54915 (US); WOLTMAN, Garry, Roland, Appleton, Wisconsin 54911 (US); CLARK, James, Joseph., JR, Appleton, Wisconsin 54914 (US)
(74) Representative: Davies, Christopher Robert
(86) International application number: PCT/US2006/034807
(87) International publication number: WO 2007/040896

(56) References cited:
- US-A1- 2003 078 552
- US-B1- 6 599 598

## Description

### FIELD OF THE INVENTION

The present invention relates to individually wrapped absorbent personal care articles, such as absorbent personal care products. More particularly, the invention relates to an improved wrapping of such products.

### BACKGROUND OF THE INVENTION

Absorbent personal care articles, such as adult incontinence products and feminine care products, have been employed to absorb urine, menses, and other body fluids. Examples of the feminine care products include sanitary napkins, panty liners, and other types of catamenial devices. The feminine care products have been used during a woman's menstrual cycle and are primarily disposable. In addition, disposable absorbent personal care articles have also been used between menstrual cycles for light incontinence purposes. Since many of these articles may be carried in a person's purse, carrying case or pocket prior to use, it is advantageous to individually wrap each article to keep it clean and sanitary. By individually wrapping each absorbent personal care article, the manufacturer can be assured that the article will not become contaminated by the contents of the user's purse, pocket, etc.

The conventional, article wrapper has included one or more layers of a thin sheet or film of thermoplastic material, such as polyethylene, and/or one or more layers of a nonwoven fibrous material. The wrapper has been folded around the absorbent personal care article, and then sealed by the use of heat and/or pressure, ultrasonics, or adhesive to form a pouch or other container. The pouch has been designed to be opened by breaking or tearing the material at or adjacent a seal in order to subsequently remove the absorbent personal care article. Conventional pouches have also been typically designed so that a soiled article can be wrapped up in the opened pouch for later disposal.

When the wrapper has been made from a polymer film, the wrapper has exhibited a number of undesirable properties. One undesirable property is the rustling and zipping sound made when the wrapper is opened. This sound undesirably communicates to others that a person is using the personal care product. In addition, the person using the product often dislikes the feel and appearance of a film material. Where the pouches have been constructed with highly breathable or highly porous materials, and the pouches have been employed to wrap or otherwise contain a soiled absorbent personal care article for disposal, it has been difficult to control malodors emanating from the soiled product. Odor control components have been added to the absorbent personal care articles or to the pouches as disclosed in US 2003/0078552 Patent Application Publication, but it has been difficult to maintain the effectiveness of the odor control components. As a result, there has been a continuing need for improved techniques and systems that can more effectively control malodors arising from soiled articles that are held in pouch materials having a high permeability to air.

### SUMMARY OF THE INVENTION

The present invention relates to a wrapped article which includes a personal care absorbent personal care article, and a wrap member which operatively encloses the absorbent personal care article. At least a major portion of the wrap member includes an outer layer, an inner layer and an intermediate layer that is sandwiched between the inner and outer layers. The intermediate layer includes an operative amount of an odor control agent. In particular aspects, the intermediate layer can include a nonwoven fibrous web, and the fibrous web can have selected fiber sizes. In another aspect, the intermediate layer of the wrap member can include a meltblown nonwoven web. In further aspects, the outer layer can include a nonwoven fibrous web having particular fiber sizes, and the inner layer can include another nonwoven fibrous web having the same or different fiber sizes.

The wrapped article of the invention can be constructed with a wrap member that includes highly breathable or highly porous materials, and the wrap member can be employed to wrap, contain or otherwise enclose an individual, absorbent personal care article. In a particular feature, the wrap member may be employed to wrap or otherwise contain a soiled personal care article for disposal. The configurations of the wrapped article can more effectively control malodors emanating from a personal care article that is held in the wrap member. In a particular aspect, the wrap member can better maintain the effectiveness of the odor control agent and can help shield the odor control material from adverse environmental effects. The wrap member can also be more readily processed during the manufacture of the wrapped article. Additionally, the wrap member can provide a soft, tactile feel while also providing a desired level of securement and containment of the odor control material. As a result, the present invention can provide an improved technique and system that can more effectively control malodors arising from articles that are held in wrap materials having a relatively high permeability to air.

### BRIEF DESCRIPTION OF THE DRAWINGS

The invention will be better understood by reference to the following description of the invention taken in conjunction with the accompanying drawings, wherein:

FIG. 1 shows a perspective view of a representative, individually wrapped article.

FIG. 2 is a partially enlarged and partially cut-away, perspective view of a representative wrapped article, shown with the wrap member in an opened state.

FIG. 3 is a cross-sectional view of a wrapped article taken along the lines indicated in FIG. 1.

FIG. 4 shows a representative, plan view of a bodyside of a representative article that can be employed with the present invention.

FIG. 5 shows a representative, plan view of a garment-side of a representative article that can be employed with the present invention.

### DETAILED DESCRIPTION OF THE INVENTION

Reference will now be made in detail to one or more configurations of the invention, at least one example of which is shown in the drawings. The configurations are provided by way of explanation of the invention, and not meant as a limitation of the invention. For example, features illustrated or described as part of one configuration embodiment may be used with another configuration to yield still a different configuration. It is intended that the present invention includes these and other modifications and variations, as come within the scope and spirit of the invention.

It should be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

As used herein, the term "nonwoven" refers to a fabric web that has a structure of individual fibers or filaments which are interlaid, but not in an identifiable repeating manner.

As used herein, the term "fiber" or "fibrous" refers to elongated individual natural or synthetic strands (as compared to a continuous film layer). Synthetic fibers are formed by passing a polymer through a forming orifice such as a die. Unless noted otherwise, the terms "fibers" or "fibrous" include discontinuous strands having a definite length and continuous strands of material, such as filaments.

As used herein, the terms "spunbond" or "spunbonded fiber" refer to fibers which are formed by extruding filaments of molten thermoplastic material from a plurality of fine, usually circular, capillaries of a spinneret, and then rapidly reducing the diameter of the extruded filaments.

As used herein, the phrases "meltblown" or "meltblown fiber" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers.

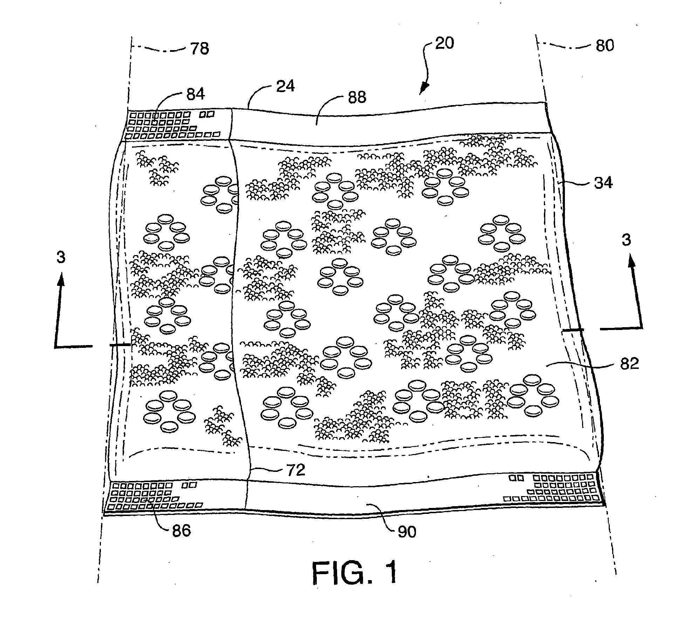

With reference to FIGs. 1 through 3, a wrapped article 20 includes a personal care absorbent personal care article 22, and a wrap member 24 which operatively encloses the absorbent personal care article 22. Desirably, the wrap member can be a separately provided component that is subsequently assembled or otherwise combined with the personal care article. At least a major portion of the wrap member 24 can include an outer layer 26, an inner layer 28 and an intermediate layer 30 that is sandwiched between the inner and outer layers. Additionally, the intermediate layer 30 can include an operative amount of an odor control agent 32. In a desired arrangement, substantially the entire wrap member 24 can include the outer, inner and intermediate layers. In particular aspects, the intermediate layer 30 can include a nonwoven fibrous web or fabric, and the fibrous web or fabric can have selected fiber sizes. In another aspect, the intermediate layer 30 of the wrap member 24 can include a meltblown nonwoven web. In further aspects, the outer layer 26 can include a nonwoven fibrous web or fabric having particular fiber sizes, and the inner layer 28 can include another nonwoven fibrous web or fabric having the same or different fiber sizes.

The wrap member 24 can include highly breathable or highly porous materials, which can be efficiently employed to hold or contain an individual, absorbent personal care article. Additionally, the wrap member may be employed to hold or contain a soiled personal care article for disposal. By incorporating its various aspects and features, alone or in combination, the invention can more effectively control malodors emanating from the personal care article that is held in the wrap member. In a particular aspect, the wrap member can better maintain the effectiveness of the odor control agent. As a result, the present invention can provide an improved technique and system that can more effectively control malodors arising from articles that are held in wrap materials having a high permeability to air.

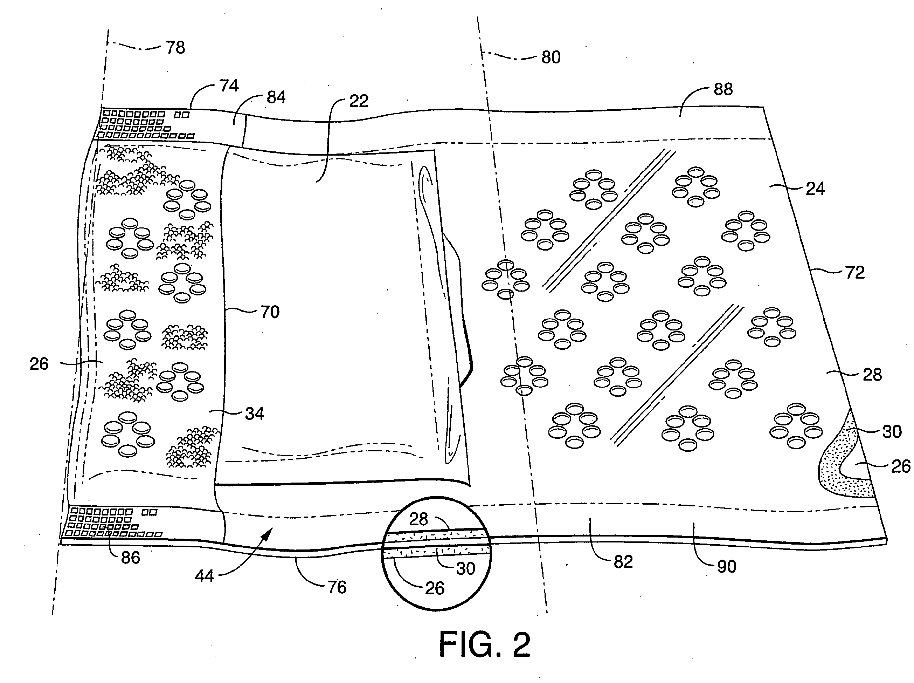

The invention is not necessarily limited to any particular configuration of the pouch 34 or other container that is formed with the wrap member 24. It is believed that the wrap member according to the invention will offer distinct advantages in any conventional pouch configuration as compared to the same pouch configuration made solely from conventional layers of polymer films or nonwoven fabrics. Various pouch configurations are known and used in the art for individually wrapping absorbent personal care articles, and any such configuration may be used in a wrapped article according to the invention. The distinctive features of the present wrap member 24 can provide a benefit to any pouch configuration. In the illustrated arrangements, for example, the pouch 34 is similar to the pouch configuration employed with POISE and KOTEX ULTRATHIN adult care pads produced by Kimberly-Clark Corporation. As representatively illustrated in FIG. 2, the wrapped article 20 can have a configuration in which each selected, personal care article 22 is individually wrapped, and the absorbent personal care article 22 is operatively carried in the wrap member 24.

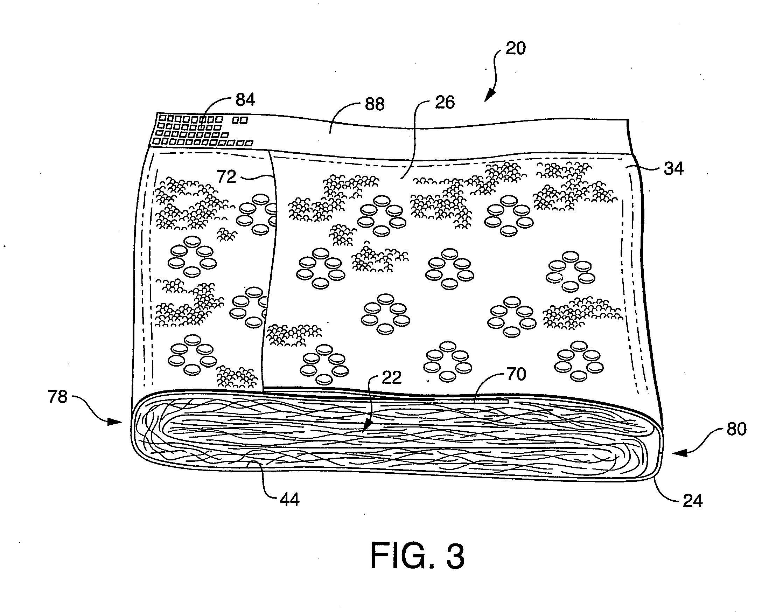

The invention is not necessarily limited to any particular type of absorbent personal care article. The absorbent personal care article 22, may, for example, be a catamenial device (such as a sanitary napkin) a panty liner, a labial pad, an incontinence pad, or any other type of absorbent personal care article which can be used to absorb menstrual fluid, urine, body fluid, body exudate or the like. For purposes of the present disclosure, the absorbent personal care article 22 may be referred to and shown as a pad product (e.g. an adult incontinence pad or a sanitary napkin or pad). The absorbent article 22 may be folded in any desired pattern to fit in the pouch 34. For example, the absorbent article can be folded into three sections to incorporate a tri-fold pattern, as representatively shown in FIG. 3.

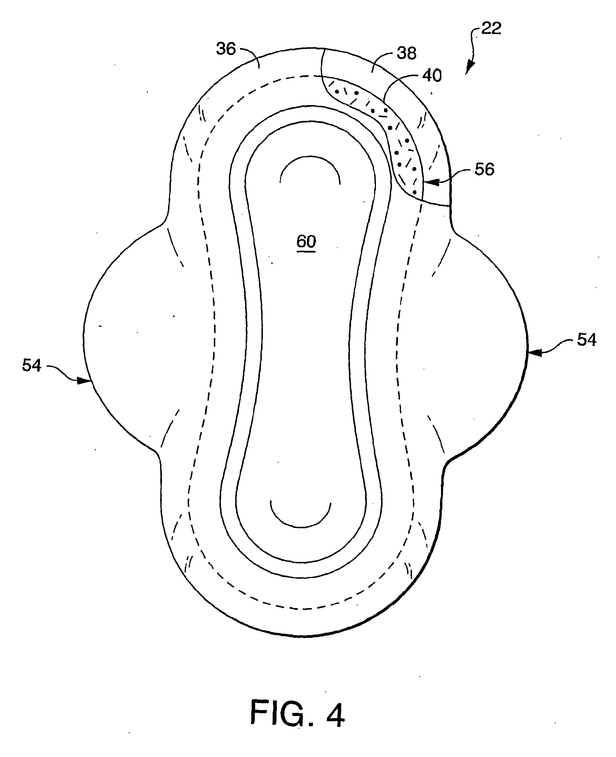

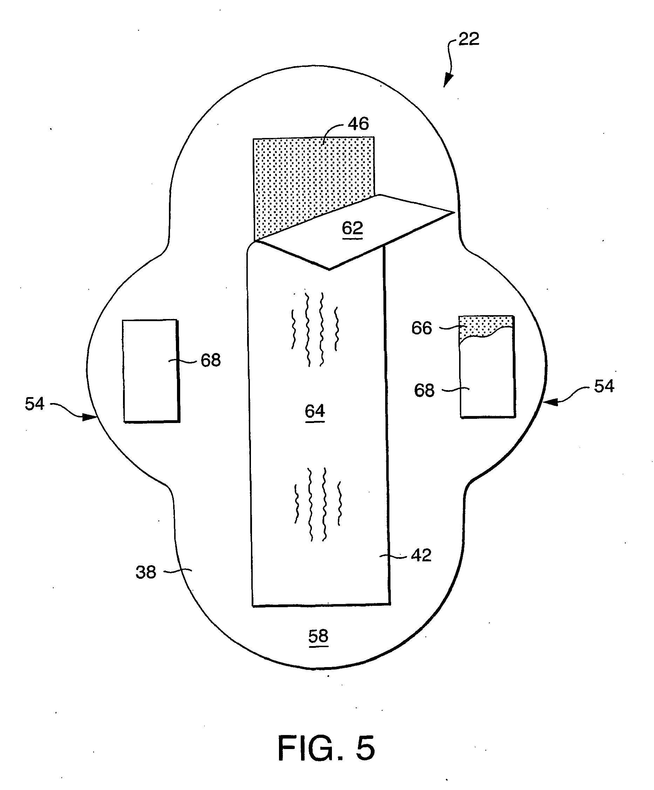

With reference to FiGs. 4 and 5, the personal care article 22 can have a generally laminated structure which includes a liquid-permeable topsheet layer 36, and a backsheet layer 38. In particular aspects, the personal care article can further include an absorbent body 40 that is positioned and held between the topsheet layer and backsheet layer. The topsheet layer 36 can comprise any operative, liquid-permeable material. For example, the topsheet layer can include a polymer film, a woven fabric, a nonwoven fabric or the like, as well as combinations thereof. The employed polymer films may be porous, or may be treated or otherwise processed to impart the desired level of liquid-permeability.

The backsheet layer 38 can comprise a polymer film, a woven fabric, a nonwoven fabric or the like, as well as combinations thereof. In desired arrangements, the backsheet layer 38 can be configured to be operatively liquid-impermeable, and can sufficiently block the movement of body-liquids through the thickness of the backsheet layer during ordinary use. In another feature, the backsheet layer 38 can be configured to be gas-permeable or "breathable". Such breathable backsheet layer materials are well known and available from commercial vendors.

The personal care article 22 may or may not include the absorbent body 40. When present in the personal care article, the absorbent body 40 can include any operative absorbent material. Examples of suitable absorbent materials can include natural fibers, synthetic fibers, woodpulp fibers, cellulosic fibers, synthetic polymer fibers, thermoplastic binder fibers, bicomponent binder fibers or the like, as well as combinations thereof. Additionally, the absorbent body 40 can include superabsorbent materials which can typically absorb and retain large amounts of aqueous liquids per unit weight of the superabsorbent material. The superabsorbent materials have very high absorbent capacities and swell to form hydrogels that are substantially water-insoluble. Such superabsorbent materials are well known and are readily available from commercial vendors.

The absorbent body 40 can be configured to provide any operative level of absorbent saturation capacity (saturation retention capacity). Typically, the absorbent capacity pertains to the ability to absorb and hold liquids, such as water, saline, urine, synthetic urine, menses or menses simulant, or the like. Particular arrangements of an adult incontinence product can be configured to provide an absorbent saturation retention capacity which is at least a minimum of about 100 grams or 150 grams of urine simulant, as determined under substantially unconstrained, free-swell conditions. The absorbent saturation capacity can alternatively be at least about 200 grams of urine simulant, and can optionally be at least about 300 grams of urine simulant to provide improved benefits. In other aspects, the product and/or its absorbent body can have a saturation capacity of up to about 1000 grams of urine simulant, or more. In a feminine care product, the absorbent saturation capacity can be a minimum of about 0.1 grams of menses simulant, or less. In other aspects, the saturation capacity can be up to a maximum of about 100 grams of menses simulant.

A suitable urine simulant for determining absorbent capacity is 0.9 wt% saline (solution composed of water and 0.9 wt% NaCl). A suitable menses simulant is composed of swine blood diluted with swine plasma to provide a hematocrit level of 35% (by volume). A suitable device for determining the hematocrit level is a HEMATOSTAT-2 system, available from Separation Technology, Inc., a business having offices located in Altamonte Springs, Florida, U.S.A. Alternatively, a substantially equivalent device or system may be employed.

The absorbent body has a peripheral edge 56. Additionally, the personal care article 22 can have a first, garment-facing side 58, and a second, bodyside 60 that is located opposite the garment-side 58. Tabs or side-panels, generally designated by 54, may be incorporated, and an individual side-panel can be configured to extend laterally from each lateral edge of the absorbent body 40 for wrapping around the crotch of the user's underwear (not shown) to protect it from staining. The tabs may also be referred to as "wings". As illustrated, each side-panel 54 may include a tab fastener, such as provided by the representatively shown adhesive strip 66 or other adhesive region, for attaching either or both side-panels in a conventional manner to an outside surface of a crotch section of the user's underwear to hold the side-panels 54 of the personal care article 22 in place around the underwear during use. The adhesive strips 66 may be covered with a removable peel strip member 68 to prevent the adhesive strips from sticking to other surfaces until ready for use.

As further illustrated, a garment-attachment mechanism, such as provided by the representatively shown region of garment adhesive 46, can be also applied to the garment side of the personal care article, thereby permitting a user to attach the personal care article to a preselected surface, such as the inside surface of the crotch of the user's underwear, to hold the personal care article 22 in place on the underwear during use. As representatively shown, the garment-attachment mechanism can include a coating or other distributed pattern of adhesive 46 (e.g:, a two-sided adhesive film or tape). The garment adhesive can be disposed on the garment-side of the backsheet 38, and can be configured to provide an operative garment-fastener. Accordingly, each individual article 22 can include a garment adhesive layer that is secured to an outward, garment-facing surface of the corresponding backsheet layer 38 of the individual personal care article. The removable layer 42 of each personal care article 22 can be removably secured to the garment-adhesive layer or other garment-attachment mechanism of each article 22. Although the adhesive garment-fastener 46 may have other sizes and shapes without departing from the scope of the present invention, in one embodiment the adhesive coating can be generally rectangular and can have a width of about three centimeters and a length of about 17 centimeters.

The wrap member 24 has a configuration which can operatively hold, contain or otherwise enclose the absorbent article. As representatively shown, the wrap member 24 can include an elongate piece of material, which can be folded and bonded into a desired pouch configuration. For example, the wrap member 24 may be an elongated, generally rectangular piece having a first end 70, an opposite second end 72, and longitudinal sides 74 and 76 extending between the ends 70 and 72. The longitudinal sides may or may not be generally parallel, as desired.

With reference to FIGs. 1-3, at least a major portion of the wrap member 24 includes the outer layer 26, the inner layer 28, and the intermediate layer 30 that is sandwiched between the inner and outer layers. Desirably, substantially the entirety of the wrap member 24 can include the outer, inner and intermediate layers. The wrap member can have the form of a laminate material which includes the outer, inner and intermediate layers, and may comprise a combination of non-woven fabric materials. The nonwoven materials can be configured to give the pouch a soft and cloth-like tactile feel, and can be configured to dampen and reduce noise associated with storing, carrying, and opening the pouch 34.

In a more particular aspect, the wrap member can include a selectively configured spunbond-meltblown-spunbond (SMS) material. A typical SMS material is described in U.S. Patent 4,041,203 to Brock et al. Other SMS products and processes are described for example in U.S. Patent 5,464,688 to Timmons et al.; U.S. Patent 5,169,706 to Collier et al.; and U.S. Patent 4,766,029 to Brock et al. Generally, an SMS material includes a meltblown web sandwiched between two exterior spunbond webs. Such SMS laminates have been available commercially for years from Kimberly-Clark Corporation under names such as SPUNGUARD and EVOLUTION. The spunbonded layers on the SMS laminates can provide durability, and the internal meltblown layer can provide porosity and additional cloth-like feel.

The outer layer 26 is appointed to provide an extending, outward-facing layer of the pouch 34 that is formed with the wrap member 24. Accordingly, when the wrap member 24 is configured in the desired pouch arrangement, the outer layer 26 is relatively further from the article 22, as compared to the inner layer 28. The inner layer 28 is appointed to provide an extending, inward-facing layer of the pouch 34 that is positioned relatively closer to the interior of the pouch. Accordingly, when the wrap member 24 is configured in the desired pouch arrangement, the inner layer 28 is relatively closer to the article 22, as compared to the outer layer 26. The intermediate layer 30 is operatively held and positioned at a location that is interposed and sandwiched between the outer layer 26 and inner layer 28.

The outer layer 26 includes a nonwoven fibrous web, and the fibrous web can have a selected basis weight. In a particular aspect, the basis weight of the outer layer can be at least a minimum of about 5 g/m². In another aspect, the basis weight can be up to a maximum of about 15 g/m². If the basis weight of the outer layer is too small the wrapper will have inadequate strength; especially, if an adhesive is used as a closure means. If the basis weight is too large, the cost of the wrapper can be too high.

The fibrous web of the outer layer 26 can also include selected fiber sizes. In particular aspects, the fibers can have a fiber diameter which is at least a minimum of about 5 micrometers (µm). The fiber diameter can alternatively be at least about 7 µm, and can optionally be at least about 9 µm to provide desired performance. In other aspects, the fiber diameter can be up to a maximum of about 100 µm, or more. The fiber diameter can alternatively be up to about 30 µm, and can optionally be up to about 25 µm to provide desired effectiveness. If the fiber size is outside the desired values, the appearance and feel of the web can differ too much from a textile fabric, like a knitted cotton fabric.

The outer layer can also include a nonwoven fibrous web which has a significant level of openness and has selected pore size values. In particular aspects, the pore size value can be at least a minimum of about 50 µm. The pore size value can alternatively be at least about 100 µm, and can optionally be at least about 200 µm or 300 µm to provide desired benefits. In other aspects, the pore size value can be up to a maximum of about 500 µm, or more. The pore size value can alternatively be up to about 450 µm, and can optionally be up to about 400 µm to provide desired effectiveness. If the pore size value is outside the desired values, the appearance and feel of the fibrous outer layer can excessively differ from that of a textile fabric, like a knitted cotton fabric.

A suitable technique for determining the pore size value is light microscopy. Micro-images are acquired using a high-resolution digital microscope system, such as a VH-6300 Digital Microscope system (which is available from the Keyence Corporation, Osaka, Japan) using a 175X magnification in a reflective mode. A substantially equivalent system of software and hardware may alternatively be employed. Fifty individual pores are identified and outlined within a view area of 27 mm² of a selected sample. The individual selected pores are the largest surface pores in the view area of the sample material. Surface pores are pores that are visible at the surface of the sample. The image analysis software that comes with the microscope is used to determine the area of the individual pores. Details regarding the operation of the microscope and the software can be found in the operational manual, which is incorporated herein by reference. The pore size is the diameter of a circle that has a circular area that equals the observed area of the measured pore. The arithmetic mean is then taken from the 50 pore diameters determined from the 50, individually measured pore areas to determine the pore size value.

In a desired aspect, the outer layer 26 of the wrap member 24 can be a spunbond nonwoven fabric. Spunbonded fibers are small diameter substantially continuous fibers that are formed by extruding a molten thermoplastic material from a plurality of fine, usually circular, capillaries of a spinnerette with the diameter of the extruded fibers then being rapidly reduced as by, for example, eductive drawing and/or other well-known spunbonding mechanisms. The production of spun-bonded nonwoven webs is described and illustrated, for example, in U.S. Patent 4,340,563 to Appel, et al., U.S. Patent 3,692,618 to Dorschner, et al., U.S. Patent 3,802,817 to Matsuki, et al., U.S. Patent 3,338,992 to Kinney, U.S. Patent 3,341,394 to Kinney, U.S. Patent 3,502,763 to Hartman, U.S. Patent 3,502,538 to Levy, U.S. Patent 3,542,615 to Dobo, et al., and U.S. Patent 5,382,400 to Pike, et al. Spunbond fibers are generally not tacky when they are deposited onto a collecting surface.

The wrap member 24 can also include an inner layer 28 provided by another nonwoven fabric (e.g. a spunbond nonwoven fibrous web) having one or more other strata of fibrous material. The inner layer 28 can be similar to or different than the outer layer 26. As a result, the parameters and features disclosed for the outer layer 26 can also be operatively applied to the inner layer 28. Thus, the various descriptors (e.g. basis weight, fiber size value and pore size value) that are disclosed as being pertinent to the outer layer 26 will also be pertinent to the inner layer 28.

Although the outer layer 26 and/or the inner layer 28 of the wrap member 24 can desirably include a spunbond nonwoven fabric, it should be appreciated that the inner and/or outer layers may include other types of fibrous webs. The layer or layers may, for example, include an extensible fibrous web, a bonded-carded web, a spunlace web, a hydroentangled web, a fibrous neck-bonded laminate, a fibrous stretch-bonded laminate or the like, as well as combinations thereof.

A further aspect of the invention can have a configuration in which the inner layer of the wrap member includes a selected amount of an absorbent material. In particular aspects, the absorbent material can provide a total absorbent capacity which is at least a minimum of about 0.3 grams of 0.9 wt% saline (solution of water and 0.9 wt% NaCl), as determined under substantially unconstrained, free-swell conditions. The total absorbent capacity can alternatively be at least about 0.5 grams of 0.9 wt% saline, and can optionally be at least about 1 gram of 0.9 wt% saline to provide desired benefits. In other aspects, the total absorbent capacity can be up to a maximum of about 5 grams of 0.9 wt% saline, or more. The total absorbent capacity can alternatively be up to about 3 grams of 0.9 wt% saline, and can optionally be up to about 2 grams of 0.9 wt% saline to provide desired effectiveness.

If the total absorbent capacity is too small, liquids may escape the wrapper when liquids are pressed out of the absorbent article 22. If the total absorbent capacity is too large, the cost of the wrap member may be excessive.

In another aspect, the inner layer 28 of the wrap member may include a superabsorbent polymer material. Any operative superabsorbent material may be employed. Such materials are well known in the art and are available from commercial vendors.

Either or both of outer layer 26 and inner layer 28 of the wrap member can be bonded or otherwise operatively attached to the intermediate layer of the wrap member. Additionally, the inner layer 28 of the wrap member 24 can be bonded or otherwise operatively attached to the outer layer of the wrap member. Any operative attachment mechanism or technique may be employed, and suitable mechanisms and techniques are well known in the art. The attachment mechanism or technique can include adhesive bonding, thermal bonding, sonic bonding, stitching, hydroentangling, mechanical attachments or the like as well as combinations thereof.

The outer layer 26 and inner layer 28 can cooperatively help to enhance the effectiveness of the wrap member 24. The inner and outer layers can, for example, help to shield and protect the intermediate layer 30, and can help to shield and protect any treatment agents that are located in the intermediate layer. Additionally, the inner and outer layers can be configured to hold one or more different treatment agents in a manner that substantially avoids an excessive degradation of the treatment agent or agents held in the intermediate layer. Where the material of the intermediate layer has an unpleasant appearance or unpleasant tactile feel, the inner and outer layers of the wrap member 24 can effectively cover the intermediate layer to present a more pleasant feel and appearance to the user.

It should be appreciated that at least one of the inner layer, outer layer and intermediate layer of the wrap member can include other, additional components or materials. For example, the inner layer 28 and/or the outer layer 26 may include a treatment material, such as a stain release agent, a softening agent, an anti-static agent, a printing enhancement agent, an adhesive release agent, or the like, as well as combinations thereof. As another example, at least the outer layer 26 of the wrap member 24 may include an operative water-repellency agent. Desirably, the adhesive release agent can be located in at least the inner layer 28 of the wrap member. The selected components or materials may optionally be incorporated with or otherwise present in any of the other layers that are included in the wrap member, as desired.

Any operative treatment material or agent may be employed. Such materials and agents are well known in the art and are available from commercial vendors. Examples of suitable softening materials and agents are disclosed in U.S. Patent Application Publication US 2004/0005457 dated January 8, 2004. Examples of suitable anti-static materials are disclosed in U.S. Patent 6,537,932 which issued March 25, 2003. Examples of printing enhancement agents are disclosed in U.S. Patent Application Publication US 2004/0121675 dated June 24, 2004. Examples of release agents are disclosed in U.S. Patent 5,814,679 which issued September 29, 1998. Examples of stain release agents and agents for water and oil repellency are disclosed in U.S. Patent 5,714,082 which issued February 3, 1998; and in U.S. Patent Application Publication US 2004/0137818 dated July 15, 2004.

The intermediate layer 30 of the wrap member 24 can include a nonwoven fibrous web, which can include selected fiber sizes. In particular aspects, the fibers can have a selected fiber diameter value, and an average fiber diameter value in the intermediate layer can be at least a minimum of about 0.5 micrometers to provide desired performance. In other aspects, the average fiber diameter value can be up to a maximum of about 6 micrometers, or more. The average fiber diameter value can alternatively be up to about 3 micrometers, and can optionally be up to about 1.5 micrometers to provide desired effectiveness.

If the fiber diameter value is too small, the intermediate layer will be difficult to make uniformly and may not have adequate strength during processing and use. If the fiber diameter value is too large, the intermediate layer will not provide adequate barrier properties and may not be able to hold an adequate amount of the odor control agent.

The intermediate layer 30 of the wrap member 24 can also include a nonwoven fibrous web, and the fibrous web can be configured to include a selected level of openness, and can be configured to have a selected pore size value. In particular aspects, the intermediate layer can have an average pore size value which is at least a minimum of about 25 micrometers. The average pore size value can alternatively be at least about 50 micrometers to provide desired performance. In other aspects, the average pore size value can be up to a maximum of about 200 micrometers, or more. The average pore size value can alternatively be up to about 150 micrometers, and can optionally be up to about 100 micrometers to provide desired effectiveness.

If the pore size is too small, the intermediate layer may not have adequate breathability. If the pore size is too large, the intermediate layer may not adequately capture and hold the odor control material (especially particulate odor control materials) during the manufacturing process, and the loss of material may excessively degrade the desired odor control function.

The nonwoven fibrous web of the intermediate layer 30 of the wrap member 24 can have a selected basis weight. In particular aspects, the basis weight can be at least a minimum of about 0.5 g/m². The basis weight can alternatively be at least about 1 g/m², and can optionally be at least about 3 g/m² to provide desired benefits. In other aspects, the basis weight can be up to a maximum of about 10 g/m², or more. The basis weight can alternatively be up to about 7 g/m², and can optionally be up to about 5 g/m² to provide desired effectiveness.

If the basis weight is too small, the intermediate layer will be difficult to make uniformly, and may not provide adequate strength during processing and use. Also, an excessively low basis weight may not adequately hold a desired amount of odor control agent. If the basis weight is too large, the intermediate layer will be too costly and prevent air flow through the web.

In a desired aspect, the intermediate layer 30 of the wrap member can include a meltblown nonwoven web. By including the meltblown nonwoven web material, the intermediate layer can more reliably and more effectively contain or hold the desired amounts of odor control agent because of the high surface area provided by the small fiber diameters in the meltblown fibrous web.

Within an individual wrap member, the total amount of the selected odor control agent can vary, depending on the agent used and the product that is to be contained in the wrap member. In particular arrangements, the amount of the odor control agent can be at least a minimum of about 0.3 mg. The amount of the odor control agent can alternatively be at least about 0.5 mg, and can optionally be at least about 0.7 mg to provide desired benefits. In other aspects, the amount of odor control agent can be up to a maximum of about 800 mg, or more. The amount of odor control agent can alternatively be up to about 700 mg, and can optionally be up to about 600 mg to provide desired effectiveness.

When employing an odor control agent that includes an operative type of activated carbon, the amount of activated carbon can be at least a minimum of about 0.5 mg. The amount of activated carbon can alternatively be at least about 0.75 mg, and can optionally be at least about 1 mg to provide desired performance. In other aspects, the amount of activated carbon can be up to a maximum of about 250 mg, or more. The amount of activated carbon can alternatively be up to about 220 mg, and can optionally be up to about 200 mg to provide desired effectiveness.

When employing an odor control agent that includes citric acid, the amount of citric acid can be at least a minimum of about 2 mg. The amount of citric acid can alternatively be at least about 3 mg, and can optionally be at least about 4 mg to provide desired benefits. In other aspects, the amount of citric acid can be up to a maximum of about 800 mg, or more. The amount of citric acid can alternatively be up to about 600 mg, and can optionally be up to about 400 mg to provide desired effectiveness.

The odor control agent can have any operative arrangement or distribution. In a desired feature, the odor control agent can desirably be substantially uniformly distributed within the intermediate layer 30 of the wrap member. In another feature, the odor control agent can be densely packed or highly concentrated in the intermediate layer. The packing-density or other concentration parameter of the odor control material should be high enough to capture the odor emanating from the soiled product.

In a particular aspect, the odor control agent or material can have a configuration in which the selected amount of odor control material is located in an odor-control area that covers at least a minimum of about 20% of the overall, total area of the outward-facing surfaces of the wrap member. The odor-control area can alternatively be at least about 25% of the outward surface area of the wrap member, and can optionally be at least about 30% of the outward surface area of the wrap member to provide desired performance. In other aspects, the odor-control area can be up to a maximum of about 100% of the outward surface area of the wrap member. The odor-control area can alternatively be up to about 90% of the outward surface area of the wrap member, and can optionally be up to about 80% of the outward surface area of the wrap member to provide desired effectiveness. A greater level of odor-control coverage can help reduce the probability that an odor will pass through the wrapper untreated. The outward surface area of the wrap member is determined when the wrap member is configured to enclose the absorbent article 22 for disposal. The areas of the outward facing surfaces of the wrap member are determined by measuring the dimensions of the plan views of the wrap-member surfaces. The area of the odor control agent is determined by measuring the projected area of the portion of the wrap member that contains the odor control agent.

The odor control agent can be configured with any operative form. For example, the odor control agent can have the form of particulates. Alternatively, the odor control agent may have the form of an applied coating. The odor control material can be incorporated into the wrap member 24 and particularly into the intermediate layer 30 by any operative technique. Suitable techniques are well known in the art, and suitable equipment systems are available from commercial vendors. For example, the odor control material can be a powder which has been incorporated into a hydrophilic, swellable, water-insoluble absorbent crosslinked polymer. The swellable polymer can be coated on the nonwoven fibrous web of the intermediate layer 30. Alternatively, the odor control material can include particulates that are adhered, bonded, entrapped, attached or otherwise operatively secured to the intermediate layer. For example, the odor control material may be placed between selected fibrous strata within the intermediate layer 30. The odor control material may optionally be sprayed onto the intermediate layer.

In a further feature, at least about 55 wt% of the odor control agent in the wrap member 24 can be contained or otherwise located in the intermediate layer 30. Desirably, at least about 60 wt% or 65 wt% of the total amount of odor control agent in the wrap member can be positioned in the intermediate layer 30, and more desirably, at least about 70 wt% or 75 wt% of the total amount of odor control agent in the wrap member is positioned in the intermediate layer. The placement of the odor control material in the intermediate layer can better shield the odor control material from environmental factors that could degrade the effectiveness of the odor control material. The presence of the inner layer 28 and the outer layers 26 can, for example, help to shield the odor control material from contaminants.

In the various configurations of the invention, the odor control agent can include any operative odor control material. Examples of suitable odor control materials can include activated carbon, cyclodextrin, activated carbon, baking soda, absorbent gelling materials, zeolites, silicas, alumina, titania, magnesia, metal oxides, ethylenediaminetetraacetic acid (EDTA), anthraquinone dyes, metal coated nanoparticles, citric acid or the like, as well as combinations thereof. Other odor control agents are described in U.S. Patent Application Publication US 2004/0122385 dated June 24, 2004; U.S. Patent Application Publication US 2004/0122387 dated June 24, 2004; U.S. Patent 4,842,593 which issued June 27, 1989; and U.S. Patent 6,455,034 which issued September 24, 2002.

Suitable materials for use as odor control agents may be, at least partially, defined by the predominate malodor(s) to be sorbed or otherwise treated. For example, the predominant malodors from fresh urine are amines. As microbial metabolism progresses over time during disposal, the nature of the malodors can progress over time during disposal, and the nature of the malodors can change from amines to ammonia and thiols. In wastes like feces and menses, the general tendency is for the predominant malodors from "young" samples to be organic acids, alcohols, amines and disulfides. The predominant malodors of "aged" samples that have undergone further anaerobic microbial degradation tend to be esters, ketones, and thiols. However, malodors can vary among such samples, as well as within samples over time, making it difficult to predict what malodors might be present for whole class of wastes. In addition, some of the malodors such as skatole and hexanoic (caproic) acid appear to predominate from a sensory perspective because the human nose is more responsive to their presence; rather than because their absolute concentrations are high when measured analytically. As mentioned earlier, the wrap member 24 can desirably have a configuration which is permeable to non-malodorous low molecular weight gases, and operatively impermeable to the majority of malodors, which have greater than three carbons in the chemical formulations.

Due to the inherent variability of the malodors to be managed from different body fluids, it is desirable to use a combination of components in the odor-controlling compositions, though a single-component odor-controlling composition can provide adequate performance when applied appropriately. For example, activated carbon is a broad-spectrum adsorbent well known in the art. Activated carbon used alone can be efficacious for controlling a variety of malodorous gases, particularly malodorous organic compounds in which the number of carbons in the formulations of those compounds increases beyond three. Activated carbon is particularly effective when those malodors can be restricted or even substantially prevented from immediately dispersing into the general environment. The desired level of restriction can be accomplished by employing an operatively sustained containment which permits sufficient time for a sufficient adsorption of the malodor.

The activated carbon may be adjusted such that adequate capacity, adequate sorption kinetics and adequate malodor retention are operatively provided at appropriate ratios. For example, activated carbon granules with diameters less than about 40 µm tend to have a relatively poor retention of adsorbed malodors. Activated carbon granules with diameters greater than about 250 µm tend to have a relatively good retention of adsorbed malodors, but tend to have relatively slower, short-term (up to four hour) adsorption kinetics, and can have some unusable sorption capacity deep within the granules. The activated carbon granules can desirably be configured to have diameters between about 40 µm and about 250 µm, and can more desirably be configured to have diameters between 80 µm and 125 µm. A representative example of a suitable activated carbon granule can include RGC 80x325 mesh size material, which is available from Westvaco Corporation of Charleston, S. C. U.S.A., and can provide desired levels of sorption kinetics, capacity and malodor retention.

Crystalline odor control materials may optionally be employed. For example, mono-, di-, and tri-carboxylic acids such as adipic, citric, maleic, malic and malonic acid, and polymeric acids such as alginic and polyacrylic acid, can be employed to help mitigate odoriferous compounds by chemical neutralization. Such acids are effective for malodors that have a polar nitrogen, such as ammonia and organic amines from urine and menses wastes. A desired odor-control agent or material can include polyacrylic acid, alginic acid, and/or citric acid. In arrangements which include an odor-control material containing activated carbon and citric acid, the ratio of activated carbon to citric acid can be from about 1:10 to about 10:1, by weight.

Metal oxides, such as aluminum, copper, magnesium, manganese, silica, titanium and zinc oxides, can be used to control odors that have a polar nitrogen, polar carboxylic acid group and/or a polar thiol group. Accordingly, suitable odor-control materials can include zinc oxide and/or silica dioxide. Some zeolites of aluminosilicate composition have similar odor controlling properties and may optionally be employed.

Granules of crystalline acid and metal oxide granules can desirably have diameters between about 40 µm and about 250 µm. The granules can more desirably have diameters between about 80 µm and about 135 µm. When acid and/or metal oxide granules are used in combination with activated carbon granules for the present invention, the diameters of the acid and metal oxide granules can desirably be configured to be, on average, as least one-half the diameter of the activated carbon granules and not greater than twice the diameter of the activated carbon granules.

The odor control agent or material employed with the article may include a material that provides an operative level of fragrance neutralization and/or fragrance masking. The fragrance neutralization and/or fragrance masking alone may not be adequate against strong malodors such as from feces, but can be effective when used in conjunction with other odor-control technologies that substantially reduce or eliminate some of the most offensive malodors. For a fragrance to be effective as part of the odor-control material, the fragrance should provide a minimal level of interference with the activity and/or capacity of sorbents and other components of the odor-control material. It is desired that any fragrance used is more persistent, lingering or "durable" than the malodors being controlled. It is also desirable that the employed fragrance is of the fragrance neutralization type.

A suitable fragrance component of the odor-control material may include one to twelve perfume raw materials (PRMs), may alternatively include two to six PRMs, and may optionally include three to four PRMS, wherein the PRMs are chosen from among aliphatic, aromatic, polycyclic and hetrocyclic chemicals and any combination thereof. The fragrance component of the odor-control material can be premixed with other components of the composition or applied separately, for example, by spraying. To control the release of the fragrance, the fragrance component may be encapsulated.

An appropriate fragrance material may, for example, include a combination of essential oils. An essential oil is a highly concentrated, volatile liquid originating from a botanical source. A typical essential oil can include a complex mixture of alcohols, aldehydes, esters, ketones, oxides, phenols and terpenes. The oils can be extracted by steam distillation, and other techniques known in the art, from a variety of plant components, including, but not limited to roots, leaves, bark, flowers and pulp. The essential oils can, for example, include rosemary oil, clove oil, ginger oil, lavender extract, chamomile extract, aloe extract, green tree extract, rose extract or the like, as well as derivatives, variations and combinations thereof.

The odor-control material may be adhered or otherwise operatively attached directly to an individual layer of the wrap member 24, or may be operatively entrapped within the intermediate layer of the wrap member by employing any operative mechanism. The odor-control material can be attached with or without the use of a binder or adhesive. For example, the odor-control material can be applied to the intermediate layer by various printing processes, prior to the assembly of the intermediate layer into the wrap member 24. Examples of suitable printing methods are disclosed in U.S. Patent 5,693,385 and U.S. Patent 5,540,916. The printing methods can, for example, adhere activated carbon material to the surface of the intermediate layer. The odor-control material can be applied to the intermediate layer prior to or during the making of the wrap member 24. For example, the odor control material can be dispersed or otherwise applied at a time during the formation of the intermediate layer when the material of the intermediate layer is still tacky.

The odor-control material may alternatively be applied to the intermediate layer prior to or during the manufacture of the wrap member 24 by employing a spray-on process to distribute and attach the adhesive binder onto appointed regions of the intermediate layer material. An example of a suitable spray-on process is disclosed in U.S. Pat. No. 2,690,415, which describes a spray-on process for adhering activated carbon to a fluid permeable material. Optionally, an odor-control material can be imbedded in a non-woven or fibrous matrix, and the matrix can be applied to or otherwise combined with the intermediate layer. An example of a suitable process is disclosed in U.S. Patent 4,289,513.

The intermediate layer 30 can be configured to include an operative amount of the odor control agent. In a desired feature, the odor control agent has been included in an amount which is sufficient to cover, hide or eliminate undesired odors from a soiled personal care article 22.

The wrap member 24 can be configured to provide the desired effectiveness while employing relatively low amounts of odor-control material. In a particular aspect, the odor-control material can have a basis weight which is less than about 25 g/m². The basis weight of the odor control material can alternatively be less than 5 g/m², and can optionally be less than about 0.5 g/m² to provide desired benefits.

The intermediate layer 30 may also include a superabsorbent material. Suitable superabsorbents are well known and available from commercial vendors. In a particular aspect, the intermediate layer may include superabsorbent particles or fibers that are blended or otherwise held in a matrix of meltblown fibers. Where the intermediate layer 30 includes superabsorbent material, the inner layer 28 of the wrap member 24 may include an operative amount of surfactant to facilitate the movement of liquids through the inner layer 28 and into the intermediate layer 30.

Another aspect of the invention can have a configuration in which the major portion of the wrap member (with the inner, outer and intermediate layers) has a selected porosity value (as determined through the thickness dimension of the wrap member). In desired arrangements, a major portion of the wrap member composite (which includes the inner, outer and intermediate layers) can have a selected breathability resistance value, as determined through the thickness dimension of the wrap member. In particular aspects, the breathability resistance can be less than about 0.04 KiloPascals * seconds per meter of thickness of the wrap member (KPa-s/m). The breathability resistance can alternatively be less than 0.03 KPa-s/m, and can optionally be less than 0.02 KPa-s/m to provide desired performance. If the breathability resistance is too high, the wrap member will not allow air flow through it and will be too costly.

The breathability resistance of the wrap member 24 can be determined by employing a textile air permeability tester, such as a breathability resistance tester. A suitable breathability resistance tester is model number *KES F8 AP1* manufactured by Kato Tech, a business having offices located at Kyoto, Japan. A substantially equivalent tester may optionally be employed.

In a representative configuration of the pouch 34, the wrap member 24 may be folded at a first fold axis such that a first end is folded towards the opposite second end and bonded along aligned longitudinal sides to define the pouch. The outer layer of fibrous material is outwardly disposed and thus defines the outer surfaces of the pouch. The second end of the wrap member can be folded at a second fold axis back over onto a front surface of the pouch to define a flap. This flap may have various lengths or shapes. For example, the flap may extend so as to be generally adjacent the first fold axis, or even extend beyond the fold axis. Optionally, the flap may extend onto the front surface of the pouch just enough to overlap the first end of the wrap member. The flap is subsequently adhered or otherwise attached relative to the front side of the pouch. For example, the longitudinal sides of the flap may be bonded with the sides of the pouch such that three, stacked repetitions of the wrap member are bonded together along the common bond seams. The flap sides are, however, bonded to the fibrous layer of the wrap member along at least a portion thereof. Any conventional process may be utilized to bond the respective flap sides and pouch sides including, for example, a thermal embossing process.

With reference to FIGs. 1-3, the wrap member 24 can be folded around the absorbent personal care article 22 such that the pouch 34 operatively holds, contains or otherwise encloses the article. The wrap member 24 can be first folded at a first fold axis 78 such that the first end 70 of the wrap member can be folded towards but spaced from the second end 72 of the wrap member. The distance between the first end 70 and second end 72 may vary depending on the desired length of a resulting flap portion 82, as further described below. The longitudinal sides of the wrap member 24 are arranged to operatively define or otherwise delimit the sides 84 and 86 of the pouch 34. The second end 72 of the wrap member can be then folded at a second fold axis 80 so as to extend back over the first end 70 and thus define the flap portion 82 that closes off the pouch 34 (e.g. FIG. 1). The flap 82 has longitudinal sides 90 and 88 that may or may not be configured to align with the wrap member sides 74 and 76 and/or the pouch sides 84 and 86. The sides of the wrap member 24 are then bonded in a conventional manner, for example, with a heat/pressure embossing roll. The flap sides 88 and 90 can be bonded to the wrap member sides 74 and 76 and pouch sides 84 and 86 in a single or multiple pass operation. It may be the case that the first end 70 of the wrap member 24 extends essentially to the second fold axis 80 and, thus, the flap sides 88 and 90 may be bonded along their entire length to the pouch sides 84 and 86.

Referring to FiGs. 1 and 3, it can be seen that the edge of the second end 72 extends across the front surface of the pouch 34. It may be desired to adhere all or a portion of this edge to the pouch surface. However, in a desirable embodiment of the pouch according to the invention, the entire edge or a selected portion of this edge between its bonded sides 88 and 90 can be left un-adhered to the pouch.

Referring to FIG. 2 in particular, it can be seen that once the absorbent personal care article 22 can be placed on the inner major surface 44 of the wrap member 24, the material can be folded at the first axis 78 and then at the second axis 80 to define the pouch 34 and flap 82. The sides of the pouch and flap are then bonded, sealed together or otherwise operatively held together with any suitable securement or closure mechanism known to those skilled in the art. Additionally, the free edge of the flap can be releasably secured to an appropriate surface of the pouch. The securement or closure mechanism can, for example, include an adhesive bond, a cohesive bond, a thermal bond, an ultrasonic bond, a snap fastener, a button fastener, a tongue-and-groove fastener (e.g. ZIPLOC fastener), zipper, a drawstring system, an interengaging mechanical fastener, hook-and-loop fastener or the like, as well as combinations thereof. Additionally, the securement or closure mechanism may have multiple components or sections, and the sections may be distributed in any operative pattern or array.

Operative securements, such as seals or bonds, can be formed by heat, heat and pressure, pressure, adhesive, ultrasonic bonding, or the like, as well as other types of bonding techniques. The employed securement mechanism can be configured to provide a "permanent" securement, where the wrap member adjacent to the seal will typically tear or break before the secured layers separate. Alternately, the securement mechanism may be configured to provide a "frangible" securement, which can be readily separated or pulled apart by the user. In a desirable embodiment of the wrap member 24, the securement between the flap sides 90 and 88 and the pouch sides 84 and 86 can be a frangible seal formed by simultaneously sealing the pouch sides and flap sides in a single sealing operation with a heated/pressure embossing roll. The temperature of the embossing roll may and the roll cylinder pressure may be adjusted accordingly to obtain the desired bond characteristics.

In the representatively shown embodiment, the consumer can open the pouch by inserting a finger between the flap 82 and front surface of the pouch 34 and then pulling the flap away from the pouch or sliding the finger side-ways to break the sealed sides of the flap. The second end 72 of the wrap member may be substantially non-adhered to the front surface of the pouch. As a result, it can be relatively easy for the consumer to insert a finger between the flap and pouch.

Those skilled in the art will recognize that the present invention is capable of many modifications and variations without departing from the scope thereof. Accordingly, the detailed description and examples set forth above are meant to be illustrative only and are not intended to limit, in any manner, the scope of the invention as set forth in the appended claims.

## Claims

1. A wrapped article comprising:
a personal care absorbent article; and
a wrap member which operatively encloses the absorbent article;
wherein
at least a major portion of the wrap member includes an outer layer, an inner layer and an intermediate layer that is sandwiched between the inner and outer layers; and
the intermediate layer includes an operative amount of an odor control agent.

2. A wrapped article as recited in claim 1 wherein the intermediate layer includes a nonwoven fibrous web having an average fiber diameter which is up to a maximum of about 6 micrometers.

3. A wrapped article as recited in claim 1 wherein the intermediate layer of the wrap member includes a meltblown nonwoven web.

4. A wrapped article as recited in claim 1 wherein the intermediate layer of the wrap member includes a meltblown nonwoven web having a basis weight of at least about 0.5 g/m².

5. A wrapped article as recited in claim 1 wherein
the outer layer includes a nonwoven fibrous web having fiber sizes with an average fiber diameter of at least about 5 micrometers; and
the inner layer includes a nonwoven fibrous web.

6. A wrapped article as recited in claim 1 wherein the odor control agent has the form of particulates.

7. A wrapped article as recited in claim 1 wherein the odor control agent has the form of an applied coating.

8. A wrapped article as recited in claim 1 wherein said major portion of the wrap member has a breathability resistance which is less than about 0.04 KPa-s/m.

9. A wrapped article as recited in claim 1 wherein the odor control agent is distributed over an odor-control area that is at least about 20% of a total outward-facing surface area of the wrap member; and the odor control agent is provided at an amount of at least about 0.2 mg.

10. A wrapped article as recited in claim 1 wherein the outer layer of the wrap member is a spunbond nonwoven fabric.

11. A wrapped article as recited in claim 1 wherein the outer layer of the wrap member is a spunbond nonwoven fabric having a basis weight of at least about 5g/m².

12. A wrapped article as recited in claim 1 wherein the outer layer of the wrap member further includes a stain release agent.

13. A wrapped article as recited in claim 1 wherein the outer layer of the wrap member further includes a softening agent.

14. A wrapped article as recited in claim 1 wherein the outer layer of the wrap member further includes a printing enhancement agent.

15. A wrapped article as recited in claim 1 wherein the inner layer of the wrap member is a spunbond nonwoven fabric.

16. A wrapped article as recited in claim 1 wherein the inner layer of the wrap member is a spunbond nonwoven fabric having a basis weight of at least about 5 g/m².

17. A wrapped article as recited in claim 1 wherein the inner layer of the wrap member includes a nonwoven fibrous web having fiber sizes with an average fiber diameter of at least about 5 micrometers.

18. A wrapped article as recited in claim 1 wherein the inner layer of the wrap member includes an absorbent material which provides a total absorbent capacity of at least a minimum of about 0.5 g of 0.9 wt% saline.

19. A wrapped article as recited in claim 1 wherein the inner layer of the wrap member includes an adhesive release agent.

20. A wrapped article as recited in claim 1 wherein at least one of the inner layer, outer layer and intermediate layer of the wrap member further includes a water-repellency agent.

## Patentansprüche

1. Ein umwickelter Artikel, welcher umfasst:
einen absorbierenden Körperpflegeartikel; und
ein Wickelteil, welches den absorbierenden Artikel wirksam umgibt;
wobei
mindestens ein größerer Teil des Wickelteils eine äußere Schicht, eine innere Schicht und eine intermediäre Schicht enthält, welche zwischen der inneren und der äußeren Schicht eingefügt ist; und
wobei die intermediäre Schicht eine wirksame Menge eines Geruchskontrollmittels enthält.

2. Ein umwickelter Artikel gemäß Anspruch 1, wobei die intermediäre Schicht ein Faservlies enthält, welches einen durchschnittlichen Faserdurchmesser hat, welcher bis zu einem Maximum von ungefähr 6 Mikrometern beträgt.

3. Ein umwickelter Artikel gemäß Anspruch 1, wobei die intermediäre Schicht des Wickelteils ein Meltblown-Vlies enthält.

4. Ein umwickelter Artikel gemäß Anspruch 1, wobei die intermediäre Schicht des Wickelteils ein Meltblown-Vlies enthält, welches eine Flächenmasse von mindestens ungefähr 0,5 g/m² aufweist.

5. Ein umwickelter Artikel gemäß Anspruch 1, wobei
die äußere Schicht ein Faservlies umfasst, welches Fasergrößen mit einem durchschnittlichen Faserdurchmesser von mindestens ungefähr 5 Mikrometern aufweist; und wobei die innere Schicht ein Faservlies enthält.

6. Ein umwickelter Artikel gemäß Anspruch 1, wobei das Geruchskontrollmittel die Form von Partikeln aufweist.

7. Ein umwickelter Artikel gemäß Anspruch 1, wobei das Geruchskontrollmittel die Form einer aufgebrachten Beschichtung aufweist.

8. Ein umwickelter Artikel gemäß Anspruch 1, wobei der größere Teil des Wickelteils eine Atmungsaktivitätsresistenz aufweist, welche weniger als ungefähr 0,04 kPa-s/m beträgt.

9. Ein umwickelter Artikel gemäß Anspruch 1, wobei das Geruchskontrollmittel über eine Geruchkontrollfläche verteilt ist, welche mindestens ungefähr 20% einer gesamten nach außen gerichteten Oberfläche des Wickelteils ausmacht; und wobei das Geruchskontrollmittel in einer Menge von mindestens ungefähr 0,2 mg bereitgestellt wird.

10. Ein umwickelter Artikel gemäß Anspruch 1, wobei die äußere Schicht des Wickelteils ein Spunbond-Vliesstoff ist.

11. Ein umwickelter Artikel gemäß Anspruch 1, wobei die äußere Schicht des Wickelteils ein Spunbond-Vliesstoff ist, welcher eine Flächenmasse von mindestens ungefähr 5 g/m² aufweist.

12. Ein umwickelter Artikel gemäß Anspruch 1, wobei die äußere Schicht des Wickelteils des Weiteren ein Farbfreisetzmittel enthält.

13. Ein umwickelter Artikel gemäß Anspruch 1, wobei die äußere Schicht des Wickelteils des Weiteren einen Weichmacher enthält.

14. Ein umwickelter Artikel gemäß Anspruch 1, wobei die äußere Schicht des Wickelteils des Weiteren ein Druckverstärkungsmittel enthält.

15. Ein umwickelter Artikel gemäß Anspruch 1, wobei die innere Schicht des Wickelteils ein Spunbond-Vliesstoff ist.

16. Ein umwickelter Artikel gemäß Anspruch 1, wobei die innere Schicht des Wickelteils ein Spunbond-Vliesstoff ist, welcher eine Flächenmasse von mindestens ungefähr 5 g/m² aufweist.

17. Ein umwickelter Artikel gemäß Anspruch 1, wobei die innere Schicht des Wickelteils ein Faservlies enthält, welches Fasergrößen mit einem durchschnittlichen Faserdurchmesser von mindestens ungefähr 5 Mikrometern aufweist.

18. Ein umwickelter Artikel gemäß Anspruch 1, wobei die innere Schicht des Wickelteils ein absorbierendes Material enthält, welches eine Gesamtaufsaugkapazität von mindestens einem Minimum von ungefähr 0,5 g einer 0,9 Gew.-% Salzlösung bietet.

19. Ein umwickelter Artikel gemäß Anspruch 1, wobei die innere Schicht des Wickelteils ein Haftmittelfreisetzmittel enthält.

20. Ein umwickelter Artikel gemäß Anspruch 1, wobei mindestens eine der inneren Schicht, der äußeren Schicht und der intermediären Schicht des Wickelteils des Weiteren ein wasserabweisendes Mittel enthält.

## Revendications

1. Article enveloppé comprenant :
un article d'hygiène personnelle absorbant ; et
une enveloppe qui enferme d'une manière opérationnelle l'article absorbant ;
dans lequel
au moins la majeure partie de l'enveloppe comprend une couche extérieure, une couche intérieure et une couche intermédiaire intercalée entre les couches intérieure et extérieure ; et
la couche intermédiaire comprend une quantité opérationnelle d'un agent de régulation des odeurs.

2. Article enveloppé selon la revendication 1, dans lequel la couche intermédiaire comprend une bande fibreuse non tissée avec un diamètre moyen de fibre qui peut aller jusqu'à un maximum d'environ six micromètres.

3. Article enveloppé selon la revendication 1, dans lequel la couche intermédiaire de l'enveloppe comprend une bande non tissée obtenue par fusion soufflage.

4. Article enveloppé selon la revendication 1, dans lequel la couche intermédiaire de l'enveloppe comprend une bande non tissée obtenue par fusion soufflage dont la masse surfacique est d'au moins environ 0,5 g/m².

5. Article enveloppé selon la revendication 1, dans lequel
la couche extérieure comprend une bande fibreuse non tissée dont les grosseurs de fibres ont un diamètre moyen de fibre d'au moins environ cinq micromètres ; et
la couche intérieure comprend une bande fibreuse non tissée.

6. Article enveloppé selon la revendication 1, dans lequel l'agent de régulation des odeurs prend la forme de particules.

7. Article enveloppé selon la revendication 1, dans lequel l'agent de régulation des odeurs prend la forme d'un revêtement appliqué.

8. Article enveloppé selon la revendication 1, dans lequel ladite majeure partie de l'enveloppe possède une résistance à la respirabilité qui est inférieure à environ 0,04 KPa·s/m.

9. Article enveloppé selon la revendication 1, dans lequel l'agent de régulation des odeurs est distribué sur une zone de régulation des odeurs qui correspond à au moins environ 20 % d'une surface totale tournée vers l'extérieur de l'enveloppe ; et l'agent de régulation des odeurs est fourni en une quantité d'au moins environ 0,2 mg.

10. Article enveloppé selon la revendication 1, dans lequel la couche extérieure de l'enveloppe est un tissu non tissé filé-lié.

11. Article enveloppé selon la revendication 1, dans lequel la couche extérieure de l'enveloppe est un tissu non tissé filé-lié dont la masse surfacique est d'au moins environ 5 g/m².

12. Article enveloppé selon la revendication 1, dans lequel la couche extérieure de l'enveloppe comprend en outre un agent de détachage.

13. Article enveloppé selon la revendication 1, dans lequel la couche extérieure de l'enveloppe comprend en outre un agent adoucissant.

14. Article enveloppé selon la revendication 1, dans lequel la couche extérieure de l'enveloppe comprend en outre un agent d'amélioration de l'impression.

15. Article enveloppé selon la revendication 1, dans lequel la couche intérieure de l'enveloppe est un tissu non tissé filé-lié.

16. Article enveloppé selon la revendication 1, dans lequel la couche intérieure de l'enveloppe est un tissu non tissé filé-lié dont la masse surfacique est d'au moins environ 5 g/m².

17. Article enveloppé selon la revendication 1, dans lequel la couche intérieure de l'enveloppe comprend une bande fibreuse non tissée dont les grosseurs de fibres ont un diamètre moyen de fibre d'au moins environ cinq micromètres.

18. Article enveloppé selon la revendication 1, dans lequel la couche intérieure de l'enveloppe comprend un matériau absorbant qui procure une capacité d'absorption totale d'au moins un minimum d'environ 0,5 g d'une solution saline de 0,9 % en poids.

19. Article enveloppé selon la revendication 1, dans lequel la couche intérieure de l'enveloppe comprend un agent antiadhésif.

20. Article enveloppé selon la revendication 1, dans lequel au moins une couche parmi la couche intérieure, la couche extérieure et la couche intermédiaire de l'enveloppe comprend en outre un agent hydrofuge.
